# EUROPEAN PATENT SPECIFICATION

(11) **EP 3 398 928 B1**
(45) Date of publication and mention of the grant of the patent: **20.01.2021**
(21) Application number: 16881647.8
(22) Date of filing: 16.12.2016
(51) Int. Cl.: C07C 41/06, C07C 43/184

(54) **METHOD FOR PRODUCING CYCLOPENTYL ALKYL ETHER COMPOUND**
VERFAHREN ZUR HERSTELLUNG EINER CYCLOPENTYLALKYLETHERVERBINDUNG
PROCÉDÉ DE PRODUCTION D'UN COMPOSÉ DE TYPE CYCLOPENTYLALKYLÉTHER

(30) Priority: 28.12.2015 JP 2015256479
(43) Date of publication of application: 07.11.2018
(73) Proprietor: Zeon Corporation, Tokyo 100-8246 (JP)
(72) Inventor: KOGOSHI, Naoto, Tokyo 100-8246 (JP); SASANUMA, Takashi, Tokyo 100-8246 (JP)
(74) Representative: Adam, Holger
(86) International application number: PCT/JP2016/087639
(87) International publication number: WO 2017/115671

(56) References cited:
- EP-A1- 3 124 463
- WO-A1-2015/147035
- JP-A- S5 925 345
- JP-A- S57 131 733
- JP-A- 2004 300 076
- JP-A- 2011 523 618
- DEROUANE, ERIC G. et al.: "Titanium-substituted zeolite beta: an efficient catalyst in the xy- functionalisation of cyclic alkenes using hydrogen peroxide in organic solvents", New Journal of Chemistry, vol. 22, no. 8, 1998, pages 797-799, XP055168374, ISSN: 1144-0546, DOI: doi:10.1039/a804478c
- UGUINA, MARIA A. et al.: "Synthesis of Titanium Silicalite-1 from an SiO2-TiO2 Cogel using a Wetness Impregnation Method", Chemical Communications, Journal of the Chemical Society, vol. 1, 1994, pages 27-28, XP055508466, ISSN: 0022-4936
- HULEA, VASILE et al.: "Oxidation of cyclopentene with H202 over B, Ga, or Al modified silicalites of MFI topology", Progress in Catalysis, vol. 9, no. 1-2, 2000, pages l3-18, XP009511388, ISSN: 1220-8698

## Description

### TECHNICAL FIELD

The present invention relates to a method for industrially advantageously producing a cyclopentyl alkyl ether compound useful as a cleaning solvent for electronic components and precision machinery components, a chemical reaction solvent, an extraction solvent, a crystallization solvent, a chromatography eluate, a solvent and a remover for electronic and electric materials, and the like.

### BACKGROUND ART

Methods for producing ethers by addition reaction between an olefin and an alcohol in the presence of a solid acid catalyst have been conventionally known.

For example, Patent Document 1 discloses a method for producing a cyclopentyl methyl ether using an acidic ion exchange resin containing 5 wt% or less of water, as a catalyst.

However, this method has required reaction in a gas phase to suppress the deterioration of the acidic ion exchange resin, and had a problem of low reaction yield.

In addition, Patent Document 2 discloses a method for producing a methyl-t-butyl ether using a crystalline aluminosilicate as a catalyst, Patent Document 3 discloses a method for producing a cyclohexyl methyl ether using a special aluminosilicate having many acid centers on an outer surface as a catalyst, and Patent Document 4 describes a method for producing a cyclohexyl methyl ether using a tungsten oxide having a specific amount of crystallization water as a catalyst.

However, Patent Documents 2 to 4 do not describe actual production of a cyclopentyl methyl ether. In addition, Patent Document 4 discloses that when High Silica Zeolite (H-ZSM-5) was used as a solid acid catalyst for a reaction between cyclohexene and methanol, the yield of the resulting methylcyclohexyl ether was only 3.7%.

In connection with the present invention, Patent Document 5 discloses a method for producing a primary alkyl-tertiary alkyl ether, in which a tertiary alcohol is reacted with a primary alcohol in the presence of a solid acid catalyst such as a pentasil type zeolite having a silica/alumina ratio of 30 to 350.

### CITATION LIST

### PATENT LITERATURE

PTL 1: WO 2003-2500, brochure (US 2005065060 A1)
PTL 2: Japanese Patent Laid-Open No. 59-25345
PTL 3: Japanese Patent Laid-Open No. 61-249945
PTL 4: Japanese Patent Laid-Open No. 5-163188
PTL 5: EP 0645360

### SUMMARY OF INVENTION

### TECHNICAL PROBLEM

The object of the present invention is to provide a method for producing a cyclopentyl alkyl ether compound by addition reaction between a cyclopentene and an alcohol in the presence of a solid acid catalyst, wherein the reaction can be carried out in a liquid phase, catalyst activity is less lowered over time (long catalyst life), and a desired cyclopentyl alkyl ether compound can be continuously produced with high reaction efficiency and long-term stability even when a large amount of raw material is fed.

### SOLUTION TO PROBLEM

The present applicant has previously reported that when a cyclopentene and an alcohol compound are reacted in the presence of an acidic zeolite having a silica/alumina ratio of 30, a cyclopentyl alkyl ether compound can be stably produced with a high reaction efficiency even in a case that a large amount of raw material is fed (WO 2015/147035 A1, brochure).

However, from following researches, it has been shown that this method has a problem that long-term continuous production is difficult because the catalytic activity is lowered over time.

Thus, as a result of further investigations, the present inventors have found that when a cyclopentene and an alcohol compound are reacted in the presence of an acidic zeolite having a silica/alumina ratio of 80 or higher, a desired cyclopentyl alkyl ether compound can be continuously produced with high reaction efficiency and long-term stability even in a case that a large amount of raw material is fed. This finding has led to the completion of the invention.

Thus, several aspects of the invention provide methods for producing a cyclopentyl alkyl ether compound of [1] to [5].
[1] A method for producing a cyclopentyl alkyl ether compound represented by formula (1): R¹-O-R² (wherein, R¹ represents an alkyl group having 1 to 10 carbon atoms that may have a substituent or a cycloalkyl group having 3 to 8 carbon atoms that may have a substituent, and R² represents a cyclopentyl group that may have a substituent), wherein a cyclopentene that may have a substituent is reacted with an alcohol compound represented by formula (2): R¹OH (wherein R¹ represents the same as described above) in the presence of an acidic zeolite having a silica/alumina ratio of 80 or higher.
[2] The method for producing the cyclopentyl alkyl ether compound according to [1], wherein the cyclopentyl alkyl ether compound represented by the formula (1) is a compound in which R¹ in the formula (1) is an alkyl group having 1 to 10 carbon atoms.
[3] The method for producing the cyclopentyl alkyl ether compound according to [1] or [2], wherein the acidic zeolite is an H-ZSM-5 type zeolite.
[4] The method for producing the cyclopentyl alkyl ether compound according to any one of [1] to [3], wherein the cyclopentene that may have a substituent is reacted with the alcohol compound represented by formula (2) in a flowing manner.
[5] The method for producing the cyclopentyl alkyl ether compound according to any one of [1] to [4], wherein a formed article of an acidic zeolite having a silica/alumina ratio of 80 or higher is used as the acidic zeolite.

### ADVANTAGEOUS EFFECTS OF INVENTION

In accordance with the production method according to one embodiment of the invention, reaction can be carried out even in a liquid phase, and a desired cyclopentyl alkyl ether compound can be continuously produced with high reaction efficiency and long-term stability even in a case that a large amount of raw material is fed.

### BRIEF DESCRIPTION OF DRAWINGS

Figure 1 is a schematic drawing illustrating one example of a reactor for carrying out the production method according to one embodiment of the invention.
Figure 2 is a graph illustrating a relationship between the concentration of the desired product in the reaction solution and the elapsed time in Examples and Comparative Examples.
Figure 3 is a graph illustrating a relationship between a rate of the concentration of the desired product in the reaction solution to its concentration at the start of the reaction and the elapsed time in Examples and Comparative Examples.

### DESCRIPTION OF EMBODIMENTS

Hereinafter, the present invention will be described in detail.

The present invention relates to a method for producing a cyclopentyl alkyl ether compound represented by formula (1): R¹-O-R², wherein a cyclopentene that may have a substituent (hereinafter referred to as "a cyclopentene" in some cases) is reacted with an alcohol compound represented by formula (2): R¹OH (hereinafter referred to as "an alcohol compound (2)" in some cases) in the presence of an acidic zeolite having a silica/alumina ratio of 80 or higher. In the present specification, the phrase "may have a substituent" means "have no substituent or have substituent".

### [Cyclopentene]

The substituent of the cyclopentene that may have a substituent used in the present invention is not particularly limited as long as it is inactive under the reaction conditions. Examples thereof include an alkyl group having 1 to 4 carbon atoms such as a methyl group, an ethyl group, an n-propyl group, an isopropyl group, an n-butyl group and a t-butyl group; an alkoxy group having 1 to 4 carbon atoms such as a methoxy group, an ethoxy group, an n-propoxy group, an isopropoxy group, an n-butoxy group and a t-butoxy group: an alkylthio group having 1 to 4 carbon atoms such as a methylthio group, an ethylthio group, an n-propylthio group and a t-butylthio group; a halogen atom such as a fluorine atom, a chlorine atom and a bromine atom; an aryl group that may have a substituent, such as a phenyl group and a 4-methylphenyl group; and the like.

Specific examples of the cyclopentene include cyclopentene, 1-methylcyclopentene, 3-methylcyclopentene, 3-ethylcyclopentene, 3-sec-butylcyclopentene, 2-t-butylcyclopentene, 1,3-dimethylcyclopentene, 3 -methoxycyclopentene, 3 -ethoxycyclopentene, 2-sec-butoxycyclopentene, 3 -t-butoxycyclopentene, 3 -methylthiocyclopentene, 3 -ethylthiocyclopentene, 2-sec-butylthiocyclopentene, 3-t-butylthiocyclopentene, 1-fluorocyclopentene, 2-chlorocyclopentene, 3 -chlorocyclopentene, 2-bromocyclopentene, 3-bromocyclopentene, 2-chloro-3-methylcyclopentene, 1-phenylcyclopentene and the like.

Above all, cyclopentene is particularly preferred from the viewpoint of availability and the like.

### [Alcohol compound (2)]

The alcohol compound (2) used in the present invention is a compound represented by formula (2): R¹OH. In formula (2), R¹ represents an alkyl group having 1 to 10 carbon atoms that may have a substituent or a cycloalkyl group having 3 to 8 carbon atoms that may have a substituent.

Examples of the alkyl group having 1 to 10 carbon atoms of the alkyl group having 1 to 10 carbon atoms that may have a substituent include a methyl group, an ethyl group, an n-propyl group, an isopropyl group, an n-butyl group, a sec-butyl group, an isobutyl group, a tert-butyl group, an n-pentyl group, a neopentyl group, an n-hexyl group, an n-heptyl group, an n-octyl group, an n-nonyl group, an n-decyl group and the like.

Examples of the substituent of the alkyl group having 1 to 10 carbon atoms that may have a substituent include an alkoxy group having 1 to 10 carbon atoms such as a methoxy group and an ethoxy group; an alkylthio group having 1 to 10 carbon atoms such as a methylthio group and an ethylthio group; a halogen atom such as a fluorine atom, a chlorine atom and a bromine atom; and the like.

Examples of the cycloalkyl group having 3 to 8 carbon atoms of the cycloalkyl group having 3 to 8 carbon atoms that may have a substituent include a cyclopropyl group, a cyclobutyl group, a cyclopentyl group, a cyclohexyl group, a cycloheptyl group, a cyclooctyl group and the like.

Examples of the substituent of the cycloalkyl group having 3 to 8 carbon atoms that may have a substituent include an alkyl group having 1 to 10 carbon atoms such as a methyl group, an ethyl group, an n-propyl group and an isopropyl group; an alkoxy group having 1 to 10 carbon atoms such as a methoxy group and an ethoxy group; an alkylthio group having 1 to 10 carbon atoms such as a methylthio group and an ethylthio group; a halogen atom such as a fluorine atom, a chlorine atom and a bromine atom; and the like.

Specific examples of the alcohol compound (2) include
an alcohol compound in which R¹ in formula (2) is an alkyl group having 1 to 10 carbon atoms, such as methanol, ethanol, n-propanol, isopropanol, n-butanol, sec-butanol, isobutanol, tert-butanol, n-pentanol and n-hexanol;
an alcohol compound in which R¹ in formula (2) is an alkyl group having 1 to 10 carbon atoms that has a substituent, including an alkoxyalkyl alcohol such as methoxymethyl alcohol, 1-methoxyethyl alcohol, 2-methoxyethyl alcohol, 2-ethoxy-tert-butyl alcohol and 2-ethoxy-n-hexyl alcohol; an alkylthioalkyl alcohol such as methylthiomethyl alcohol, 1-methylthioethyl alcohol, 2-methylthio-tert-butyl alcohol, 3-methylthio-n-butyl alcohol and 4-methylthio-n-hexyl alcohol; and a halogenated alkyl alcohol such as chloromethyl alcohol, bromomethyl alcohol, 1-chloroethyl alcohol, 2-chloro-n-propyl alcohol, 2-bromo-tert-butyl alcohol, 2-bromo-n-butyl alcohol and 2-chloro-n-hexyl alcohol;
an alcohol compound in which R¹ in formula (2) is a cycloalkyl group having 3 to 8 carbon atoms, such as cyclopropyl alcohol, cyclobutyl alcohol, cyclopentyl alcohol, cyclohexyl alcohol, cycloheptyl alcohol and cyclooctyl alcohol;
an alcohol compound in which R¹ in formula (2) is a cycloalkyl group having 3 to 8 carbon atoms that has a substituent, such as 2-chlorocyclopentyl alcohol, 4-methoxycyclohexyl alcohol and 3-methylthio cycloheptyl alcohol; and the like.

Above all, preferably the alcohol compound in which R¹ in formula (2) is an alkyl group having 1 to 10 carbon atoms or a cycloalkyl group having 3 to 8 carbon atoms is used, and more preferably the alcohol compound in which R¹ is an alkyl group having 1 to 10 carbon atoms is used, because the effects of the present invention can be more easily obtained, in the present invention.

### [Acidic zeolite]

In the present invention, an acidic zeolite having a silica/alumina ratio of 80 or higher (hereinafter simply referred to as "acidic zeolite" in some cases) is used as a reaction catalyst (solid acid catalyst).

The silica/alumina ratio is preferably 80 to 300, and more preferably 80 to 180 from the viewpoint of capability of obtaining good catalytic activity.

Since catalytic activity of such an acidic zeolite is less lowered over time and its catalyst life is long, the desired product can be industrially-advantageously obtained with high reaction efficiency and long-term stability.

The zeolite includes SiO₄ tetrahedron and AlO₄ tetrahedron, and there are many known types based on difference in bonding manner of each tetrahedron or the like. In addition, the zeolite has a three-dimensional skeleton structure and forms cavities (pores) in its lattice. The size and shape of this pore vary depending on the type of the zeolite, some types thereof have pore diameters of 3 to 12 angstroms and one- to three-dimensional pore shapes.

The zeolite can be produced by e.g. a process in which a silica source (water glass, sodium silicate, etc.) and an alumina source (aluminum hydroxide, sodium aluminate) are mixed, a template agent (seed crystal of the zeolite, etc.) is added as necessary, and the pH is adjusted, which is subsequently subjected to hydrothermal synthesis. In this case, a zeolite having a silica/alumina ratio of 80 or higher can be obtained by adjusting the molar ratio of the silica source and the alumina source.

The zeolite has ion exchange ability and normally has alkali metal ions such as Na and K in its skeleton, and the ions can be easily exchanged by bringing it into contact with various cations.

An acidic zeolite is a zeolite having an H⁺ group or a Lewis acid site on its surface.

The acidic zeolite used in the present invention is preferably that obtained by converting a beta-type zeolite, a faujasite-type zeolite, a mordenite-type zeolite, an L-type zeolite, a Y-type zeolite, an omega-type zeolite, a ZSM-5-type zeolite, a ferrierite-type zeolite or the like into an H type zeolite by the following method or the like, and more preferably that obtained by converting the ZSM-5-type zeolite into the H-type zeolite (H-ZSM-5 type).

The H-type acidic zeolite can be obtained e.g. by a process in which a zeolite is converted into an ammonium ion-type zeolite by bringing the zeolite into contact with an aqueous solution of ammonium ion (aqueous solution of NH₄Cl, NH₄NO₃, etc.), and then this is calcined at 300°C or higher to remove an ammonia. Also, it can be obtained by bringing a zeolite into contact with a strong acid such as hydrochloric acid to directly exchange the ions with H ions.

Also, a zeolite commercially available as an H-type acidic zeolite can be used as is.

The acidic zeolite used in the present invention may be a powder or a formed article, but a formed acidic zeolite (formed article of zeolite) is preferred from the viewpoint of handleability.

Particularly, in the reaction in a flowing manner, it is desirable to use a formed acidic zeolite (formed article of zeolite) from the viewpoint of pressure loss and the like.

The formed article of the acidic zeolite used in the present invention may be an article obtained by forming any of a hydrothermally-synthesized product, a dried product, a calcined product or an ion-exchanged product. When forming the zeolite, a known method such as extrusion, compression, tableting, flow, rolling and spraying can be adopted. By such a forming method, the zeolite can be formed into a desired shape, e.g. a spherical (granular) shape, a cylindrical (pellet) shape, a slab shape, a ring shape, a clover shape, a honeycomb shape and the like. For example, when a pellet-shaped product is required, a method such as extrusion and tableting can be adopted, and when a particulate-shaped product like a catalyst for a fluidized bed is required, a method such as spray drying can be adopted.

As the zeolite to be formed, a zeolite having a primary particle diameter of normally 5 µm or less, preferably 1 µm or less is used.

The size of the formed article is not particularly limited. Examples of the pellet-shaped product include a cylindrical pellet having a diameter of 0.5 to 5 mm and a height of 0.5 to 5 mm, a flat disc-shaped pellet having a diameter of 0.5 to 5 mm, and the like.

Examples of the formed article of the zeolite include a product formed by mixing a zeolite powder and a binder (hereinafter referred to as "zeolite-binder formed article" in some cases) and a product formed without using a binder component (hereinafter referred to as "binderless zeolite formed article" in some cases).

The binder used for producing the former zeolite-binder formed article is exemplified by an inorganic oxide such as an alumina/silica/clay. In addition, if necessary, the zeolite can be formed by adding polyvinyl alcohol, methyl cellulose, polyethylene oxide, a wax and the like.

When using a zeolite-binder formed article, a shape and a size of the formed article, and morphological characteristics such as mesopore and macropore volumes and their distributions are controlled to some extent to reduce a pressure loss, and at the same time, a mass-transfer rate in the formed article can be increased to achieve a high efficiency of catalyst utilization.

Examples of the method for obtaining the latter binderless zeolite formed article include a method in which a dry gel powder as a precursor is compressed on a disk, and then crystallized, a method in which silicon and aluminum are eliminated from the zeolite particle previously synthesized by a post-synthesis method such as a sodium hydroxide aqueous solution treatment and hydrothermal treatment, a method in which, from zeolite particle obtained by hydrothermal synthesis in the coexistence of carbon black and polystyrene particle, the coexisting particles are removed by calcining, a method in which an alkali metal and an organic structure-directing agent (SDA) are impregnated and supported in a silica formed article as a silicon source and crystallized under a pressurized water vapor atmosphere, and the like (see Journal of the Surface Science Society of Japan, 19,558 (1998), Adv. Mater., 8, 759 (1996), Chem. Lett., 25, 403 (1996), Zeolite, vol. 29, no. 2, 55-61, Japanese Patent Laid-Open No. 2001-58817, Bull. Chem. Soc. Jpn., 80, 1075 (2007), Surv. Asia, 14, 116 (2010), etc.).

When using a binderless zeolite formed article, it is capable to obtain not only an effect obtained in the case of using the zeolite-binder formed article, but also an effect of avoiding problems such as buried zeolite into a binder component, lowered efficiency due to diluting action, and a side reaction with an inorganic binder.

In addition, in the present invention, a formed article commercially available as an acidic zeolite formed article can be used as is.

The catalytic activity of the acidic zeolite used in the present invention is not decreased for a long term. Specifically, when the concentration of the desired product in the reaction solution at the initial phase of the reaction is taken to be 100, the period during which the concentration of the desired product in the reaction solution can be kept at 80% or higher is normally 500 hours or longer depending on the reaction method, the reaction scale, and the like. That is, the zeolite can be continuously used normally for 500 hours or longer without exchange of the catalyst or the like.

Note that the catalyst after use can be reused by being activated by a conventionally known method.

### [Production method]

The present invention is a method for producing a cyclopentyl alkyl ether compound by reacting the cyclopentene and the alcohol compound (2) in contact with each other in the presence of an acidic zeolite.

The reaction method is not particularly limited. For example, a method in which a mixture of the cyclopentene and the alcohol compound (2) (hereinafter also referred to as "mixture") is put into a sealed reactor, to which an acidic zeolite is further added, and the whole content is stirred (batch type), a method in which an acidic zeolite is charged in a column and a mixture is allowed to flow through the column (hereinafter referred to as "reaction column") (flowing type), or the like can be used.

Above all, the flowing type is preferred from the viewpoints of a working efficiency and a capability of continuously producing the desired product over a long term.

To prepare the mixture, it suffices that the cyclopentene and the alcohol compound (2) are mixed in a predetermined ratio. In this case, a process in which a mixture solution of the cyclopentene and the alcohol compound (2) is previously prepared, stored in a tank, and fed from the tank to a reaction column can be adopted, or a process in which the cyclopentene and the alcohol compound (2) are separately stored in different tanks, from which the cyclopentene and the alcohol compound (2) are separately fed, and they are mixed immediately before allowing them to flow through the reaction column and fed can be adopted.

When the batch type is adopted, predetermined amounts of the acidic zeolite, the cyclopentene and the alcohol compound (2) are added to a reactor, and the reaction mixture is stirred at a predetermined temperature and a predetermined pressure. In this case, the acidic zeolite is used in an amount of normally 0.01 to 200 parts by mass, preferably 0.1 to 150 parts by mass, and more preferably 1 to 100 parts by mass based on 100 parts by mass of the cyclopentene.

The reaction temperature is normally 50 to 250°C, and preferably 80 to 200°C, and the reaction pressure is in a range normally from normal pressure (1013 hPa, the same applies to the following.) to 10 MPa, preferably from normal pressure to 5 MPa depending on the reaction temperature and the like.

The reaction time is normally 0.5 to 24 hours, and preferably 1 to 10 hours depending on the reaction scale and the like.

The reaction is preferably carried out under an inert atmosphere such as nitrogen.

When the flowing type is adopted, the mixture is allowed to flow through the reaction column filled with the acidic zeolite. In this case, it is preferred that a column having a heating device is used and the mixture is allowed to flow through a reaction column heated to a predetermined temperature (reaction temperature).

In accordance with this method, catalytic activity is less lowered over time, thus the reaction can be continuously carried out with long-term stability without frequent exchange and activation of the catalyst.

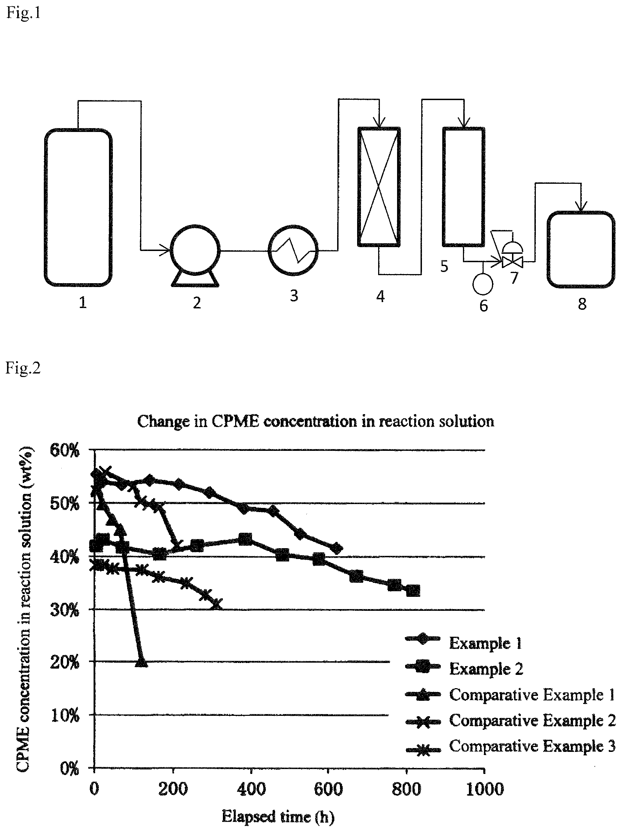

An example of a more specific method to be practiced in the flowing manner is shown in Figure 1. In Figure 1, 1 represents a raw material (mixture of the cyclopentene and the alcohol compound (2)) tank, 2 represents a liquid feeding pump, 3 represents a preheater, 4 represents a reaction column, 5 represents a cooling pipe, 6 represents a manometer, 7 represents a back pressure valve, and 8 represents a reaction liquid tank.

Note that the reaction column 4 may be used alone, but if a plurality of reaction columns are used in combination, a conversion ratio of the cyclopentene [or alcohol compound (2)] can be further improved.

The size of the column to be used is not particularly limited, and columns having various sizes can be selected depending on the reaction scale. When a plurality of reaction columns are used in combination, the types of acidic zeolite charged in respective columns may be either identical to or different from each other.

The method for allowing the mixture to flow through the reaction column filled with the acidic zeolite may be either a downflow type in which the mixture flows from an upper part of the reaction column, or an upflow type in which the mixture flows from a lower part of the reaction column. From the viewpoint of capability of obtaining a desired product with higher conversion ratio and selectivity, the downflow type is preferred. In addition, when the mixture is allowed to flow through a reaction column filled with the acidic zeolite, the mixture may be in a gas state, in a liquid state, or in a gas/liquid-mixed state.

The pressure at which the mixture passes through the reaction column is in a range normally from normal pressure to 10 MPa, preferably from normal pressure to 5 MPa, and more preferably from normal pressure to 3 MPa at the inlet of the reaction column. When the zeolite formed article is used as the catalyst, the operation can be performed at a lower pressure than in a case of using a powder catalyst.

The reaction temperature (temperature in the reaction column) is normally 50 to 200°C, and preferably 80 to 180°C.

The proportion of the cyclopentene and the alcohol compound (2) to be used is not particularly limited. Since the time for heating the mixture is short in the case of the flowing type, the cyclopentene is not polymerized, but on the other hand, use of an excessive amount of alcohol compound (2) is not preferred, because there is a possibility that an amount of by-products of the dialkyl ether is increased. Specifically, the molar ratio of (a cyclopentene)/(alcohol compound (2)) is normally 1/5 to 20/1, preferably 1/4 to 10/1, more preferably 1/3 to 5/1, and more preferably 1/3 to 3/1.

The space velocity of the cyclopentene and alcohol compound (2) passing through the reaction column [the value (hr⁻¹) representing how many times the volume of the catalyst volume is treated per unit time] is normally 0.01 to 100 hr⁻¹, and preferably 0.1 to 30 hr⁻¹.

In addition, when a plurality of reaction columns are used, the reaction temperature, the flow rate and the like can be changed for each reaction column.

In any method, the reaction can be carried out in the absence of a solvent, and also carried out in an inert solvent which dissolves the raw material cyclopentene and does not mix with water.

Examples of the solvent to be used include aliphatic saturated hydrocarbons such as n-butane, n-pentane, n-hexane, n-heptane, n-octane, n-nonane and n-decane: aromatic hydrocarbons such as benzene, toluene, ethylbenzene, xylene, anisole, cumene and nitrobenzene; alicyclic saturated hydrocarbons such as cyclopentane, alkyl-substituted cyclopentanes, alkoxy-substituted cyclopentanes, nitro-substituted cyclopentanes, cyclohexane, alkyl-substituted cyclohexanes, alkoxy-substituted cyclohexanes, nitro-substituted cyclohexanes, cycloheptane, alkyl-substituted cycloheptanes, alkoxy-substituted cycloheptanes, nitro-substituted cycloheptanes, cyclooctane, alkyl-substituted cyclooctanes, alkoxy-substituted cyclooctanes and nitro-substituted cyclooctanes; nitrogen, argon, air, helium, and the like.

The amount of the solvent to be used is not particularly limited, and the amount can be arbitrarily selected unless the reaction is inhibited. When a solvent is used, its amount is normally 10 to 90 vol%, and preferably 20 to 80 vol% based on the total amount of the reaction solution.

In any method, after completion of the reaction, the desired cyclopentyl alkyl ether compound can be isolated by a conventional separation/purification method such as solvent extraction and distillation of the reaction solution. The distillation may be carried out several times.

As the distillation apparatus, e.g. a known distillation apparatus such as a continuous rectification device having a rectification column can be used.

Also, a method in which the mixture solution is allowed to flow through a reaction column filled with the acidic zeolite, then the resulting reaction solution is passed through the reaction column again, and then continuously distilled e.g. by a distillation apparatus filled with Raschig ring, can be adopted. In accordance with this method, the unreacted cyclopentene and alcohol compound (2) can be returned to the reaction column and subjected to the reaction again, and thus the desired product can be obtained with a higher conversion ratio.

In accordance with the present invention, the desired cyclopentyl alkyl ether compound represented by formula (1): R¹-O-R² can be industrially-advantageously and continuously produced with high reaction efficiency and long-term stability even when a large amount of raw material is fed, by using the acidic zeolite having a silica/alumina ratio of 80 or higher as a solid acid catalyst.

Specific examples of the cyclopentyl alkyl ether compound obtained by the production method according to the invention include cyclopentyl methyl ether, cyclopentyl ethyl ether, cyclopentyl n-propyl ether, cyclopentyl isopropyl ether, cyclopentyl n-butyl ether, cyclopentyl tert-butyl ether, cyclopentyl n-pentyl ether, cyclopentyl n-hexyl ether, cyclopentyl methoxymethyl ether, cyclopentyl 2-methoxyethyl ether, cyclopentyl methylthiomethyl ether, cyclopentyl chloromethyl ether, cyclopentyl cyclohexyl ether and cyclopentyl 2-chlorocyclohexyl ether;
1-methylcyclopentyl methyl ether, 3-methylcyclopentyl methyl ether, 3-ethylcyclopentyl methyl ether, 1,3-dimethylcyclopentyl methyl ether, 3-methoxycyclopentyl ethyl ether, 3-methylthiocyclopentyl ethyl ether, 1-fluorocyclopentyl methyl ether, 2-chlorocyclopentyl n-pentyl ether, 3-bromocyclopentyl n-hexyl ether, 3-chlorocyclopentyl 2-methoxyethyl ether and 1-phenylcyclopentyl methyl ether; and the like, corresponding to the raw materials to be used.

### EXAMPLES

The invention is further described below by way of Examples. Note that the invention is not limited to the following Examples.

The content of each compound was measured by the following apparatus under the following conditions.
- Apparatus: GC-2010 manufactured by Shimadzu Corporation
- Column: DB-WAX (length: 30 m, inner diameter: 0.25 mm, film thickness: 0.25 µm)
- Column temperature: The temperature is maintained at 40°C for 10 minutes, then raised to 230°C at a rate of 10°C/min, and maintained at the same temperature for 1 minute.
- Inlet temperature: 200°C
- Carrier gas: Nitrogen (flow volume per minute: 0.7 ml)
- Detector: FID
- Detector temperature: 250°C

In the following examples and comparative examples, a powder of H-ZSM-5 type zeolite prepared so as to have a predetermined silica/alumina ratio was used as a solid acid catalyst (catalyst), alumina was used as a binder, and a formed article that had been formed and calcined into a cylindrical shape having a diameter of 2.2 mm and a height of 5 mm (manufactured by JGC Catalysts and Chemicals Ltd.) was used.

### (Example 1)

The following experiment was carried out using a reactor shown in Figure 1.

In a reaction column 4, a catalyst having a silica/alumina ratio of 80 was charged in a bulk volume of 100 ml. A raw material tank 1 was filled with a mixture of cyclopentene and methanol (weight ratio 68:32 (molar ratio 1:1)).

The raw material tank 1 was pressurized to 0.2 MPa with nitrogen to feed the raw material mixture solution, the gas in the liquid feeding pump 2, the preheater 3, the reaction column 4 and the cooling pipe 5 was replaced with the raw material mixture solution, and then a preset pressure of the back pressure valve 7 was raised to temporarily stop the flow of the liquid.

The liquid feeding pump 2 was operated at a flow rate of 5 ml/min, and the back pressure valve 7 was adjusted so that the instruction value of the manometer 6 was 2.8 MPa. Subsequently, the preheater 3 and the reaction column 4 were heated to 145°C, and the reaction solution flowing out from the reaction column 4 was cooled to 0°C by a cooling pipe 5 and collected in the reaction liquid tank 8.

The time at which the temperatures of the preheater 3 and the reaction column 4 reached the predetermined temperature 145°C was taken to be a starting time of the reaction, the liquid at the outlet of the back pressure valve 7 was sampled, and the concentration of the produced cyclopentyl methyl ether (CPME) as a desired product in the reaction solution (initial concentration) was measured by gas chromatography. Furthermore, the concentration of the CPME at each elapsed time was appropriately measured to calculate a ratio to the initial concentration (initial ratio). After 621 hours when the initial ratio reached 75%, the reaction was terminated.

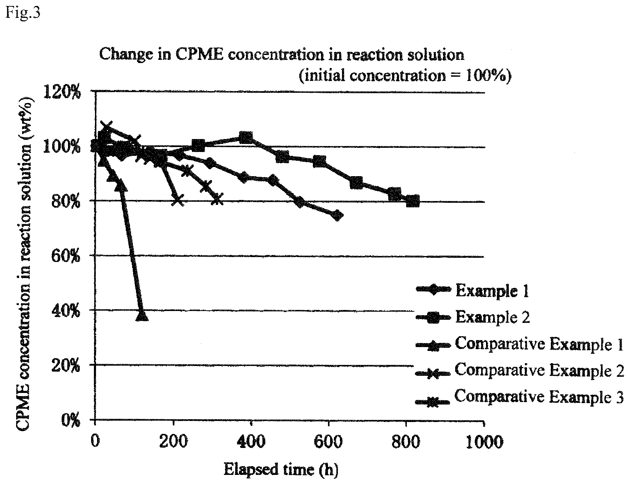

The concentration of the CPME in the reaction solution (%) and the ratio of the concentration (wt%) of the CPME to the initial concentration (%) after each elapsed time are shown in the following Table 1. Furthermore, these results are shown in the graphs of Figures 2 and 3.

In the graph of Figure 2, the ordinate represents the concentration (wt%) of the CPME and the abscissa represents the elapsed time (time). In the graph of Figure 3, the ordinate represents the ratio of the concentration of the CPME to the initial concentration (%), and the abscissa represents the elapsed time (time).

### (Example 2)

The reaction was carried out in the same manner as in Example 1, except that the silica/alumina ratio of the solid acid catalyst was changed from 80 to 180 in Example 1. After 816.5 hours when the initial ratio reached 80%, the reaction was terminated. The results are shown in the following Table 1 and graphs of Figures 2 and 3.

### (Comparative Example 1)

The reaction was carried out in the same manner as in Example 1, except that the silica/alumina ratio of the solid acid catalyst was changed from 80 to 30 in Example 1. After 119 hours when the initial ratio reached 38%, the reaction was terminated. The results are shown in the following Table 1 and graphs of Figures 2 and 3.

### (Comparative Example 2)

The reaction was carried out in the same manner as in Example 1, except that the silica/alumina ratio of the solid acid catalyst was changed from 80 to 50 in Example 1. After 209 hours when the initial ratio reached 80%, the reaction was terminated. The results are shown in Table 1 and the graphs of Figures 2 and 3.

### (Comparative Example 3)

The reaction was carried out in the same manner as in Example 1, except that the silica/alumina ratio of the solid acid catalyst was changed from 80 to 30, the weight ratio of the mixture of the cyclopentene and methanol was changed from 68:32 (molar ratio 1:1) to 41:59 (molar ratio 1:3), the preset temperature of the preheater 3 and the reaction column 4 was changed from 145°C to 150°C, the instruction value of the manometer 6 was changed from 2.8 MPa to 2.5 MPa in Example 1. After 311 hours when the initial ratio reached 81 %, the reaction was terminated. The results are shown in Table 1 and the graphs of Figures 2 and 3.

**TABLE 1**

| | | | | | | | | | | | | |
|---|---|---|---|---|---|---|---|---|---|---|---|---|
| Example 1 | Elapsed time (h) | 5 | 21 | 67.5 | 140 | 213.5 | 292 | 380 | 454.5 | 525 | 621 | |
| | CPME concentration (wt%) | 55% | 54% | 53% | 54% | 54% | 52% | 49% | 49% | 44% | 42% | |
| | Initial ratio of CPME concentration (%) | 100% | 98% | 97% | 98% | 97% | 94% | 89% | 88% | 80% | 75% | |
| Example 2 | Elapsed time (h) | 5 | 22 | 71 | 166 | 261.5 | 384.5 | 479.5 | 575 | 671 | 769 | 816.5 |
| | CPME concentration (wt%) | 42% | 43% | 42% | 40% | 42% | 43% | 40% | 40% | 36% | 35% | 34% |
| | Initial ratio of CPME concentration (%) | 100% | 103% | 100% | 97% | 100% | 103% | 96% | 95% | 87% | 83% | 80% |
| Comparative Example 1 | Elapsed time (h) | 5 | 21 | 45 | 65 | 119 | | | | | | |
| | CPME concentration (wt%) | 53% | 50% | 47% | 45% | 20% | | | | | | |
| | Initial ratio of CPME concentration (%) | 100% | 95% | 89% | 86% | 38% | | | | | | |
| Comparative Example 2 | Elapsed time (h) | 6 | 27 | 99 | 116.5 | 138 | 166 | 209 | | | | |
| | CPME concentration (wt%) | 52% | 56% | 53% | 50% | 50% | 49% | 42% | | | | |
| | Initial ratio of CPME concentration (%) | 100% | 107% | 102% | 96% | 95% | 94% | 80% | | | | |
| Comparative Example 3 | Elapsed time (h) | 2.5 | 21.5 | 46 | 121 | 163 | 234 | 282 | 311 | | | |
| | CPME concentration (wt%) | 38% | 38% | 38% | 37% | 36% | 35% | 33% | 31% | | | |
| | Initial ratio of CPME concentration (%) | 100% | 100% | 98% | 98% | 94% | 91% | 85% | 81% | | | |

Table 1 and Figures 2 and 3 show that a time-course decrease of the CPME concentration in the obtained reaction solution is smaller in the case of when using the acidic zeolite having the silica/alumina ratio of 80 or higher as a solid acid catalyst (Examples 1 and 2), compared to the case of using the acidic zeolite having the silica/alumina ratio of 80 or lower (Comparative Examples 1 to 3).

In Example 1, even after 525 hours, the desired product can be obtained in a yield of 80% of that at the start of the reaction, and also even after 621 hours, the desired product can be obtained in a yield of 42 wt%.

In Example 2, even after 575 hours, the desired product can be obtained in almost the same yield as that at the start of the reaction, and also even after 800 hours, the desired product can be obtained in a yield of 80% of that at the start of the reaction.

On the other hand, in Comparative Example 1, at the start of the reaction, the desired product can be obtained in the same yield as that in Examples, but after 119 hours, the concentration of the desired product decreases to 38% of the initial concentration.

Also in Comparative Example 2, after 209 hours, the concentration of the desired product decreases to 80% of the initial concentration.

In Comparative Example 3, the initial concentration is as low as 38 wt%, and furthermore, after 311 hours, the concentration of the desired product decreases to 81% of the initial concentration.

From the above results, it can be seen that, the decrease in the activity of the catalyst to be used is small (the catalyst life is long) in the cases of Examples compared to the cases of Comparative Examples, and thus the desired product can be stably obtained for a long term.

### REFERENCE SIGNS LIST

- 1: Raw material tank
- 2: Liquid feeding pump
- 3: Preheater
- 4: Reaction column
- 5: Cooling pipe
- 6: Manometer
- 7: Back pressure valve
- 8: Reaction liquid tank

## Claims

1. A method for producing a cyclopentyl alkyl ether compound represented by formula (1): R¹-O-R² wherein R¹ represents an alkyl group having 1 to 10 carbon atoms that may have a substituent or a cycloalkyl group having 3 to 8 carbon atoms that may have a substituent, and R² represents a cyclopentyl group that may have a substituent, wherein a cyclopentene that may have a substituent is reacted with an alcohol compound represented by formula (2): R¹OH wherein R¹ represents the same as described above in the presence of an acidic zeolite **characterized in that** the acidic zeolite has a silica/alumina ratio of 80 or higher.

2. The method for producing the cyclopentyl alkyl ether compound according to claim 1, **characterized in that** the cyclopentyl alkyl ether compound represented by the formula (1) is a compound in which R¹ in the formula (1) is an alkyl group having 1 to 10 carbon atoms.

3. The method for producing the cyclopentyl alkyl ether compound according to claim 1 or 2, **characterized in that** the acidic zeolite is an H-ZSM-5 type zeolite.

4. The method for producing the cyclopentyl alkyl ether compound according to any one of claims 1 to 3, **characterized in that** the cyclopentene that may have a substituent is reacted with the alcohol compound represented by formula (2) in a flowing manner.

5. The method for producing the cyclopentyl alkyl ether compound according to any one of claims 1 to 4, **characterized in that** the acidic zeolite is a formed article.

## Patentansprüche

1. Verfahren zur Herstellung einer Cyclopentylalkylether-Verbindung, die durch Formel (1): R¹-O-R² dargestellt wird, worin R¹ eine Alkylgruppe mit 1 bis 10 Kohlenstoffatom(en), die einen Substituenten haben kann, oder eine Cycloalkylgruppe mit 3 bis 8 Kohlenstoffatomen, die einen Substituenten haben kann, darstellt, und R² eine Cyclopentylgruppe, die einen Substituenten haben kann, darstellt,
wobei ein Cyclopenten, das einen Substituenten haben kann, mit einer Alkoholverbindung, die durch Formel (2) : R¹OH dargestellt wird, worin R¹ dasselbe, wie oben beschrieben, darstellt, in Gegenwart eines sauren Zeolithen umgesetzt wird,
**dadurch gekennzeichnet, dass** der saure Zeolith ein Siliciumdioxid/Aluminiumoxid-Verhältnis von 80 oder höher hat.

2. Verfahren zur Herstellung einer Cyclopentylalkylether-Verbindung nach Anspruch 1, **dadurch gekennzeichnet, dass** die durch die Formel (1) dargestellte Cyclopentylalkylether-Verbindung eine Verbindung ist, in der R¹ in der Formel (1) eine Alkylgruppe mit 1 bis 10 Kohlenstoffatom(en) ist.

3. Verfahren zur Herstellung einer Cyclopentylalkylether-Verbindung nach Anspruch 1 oder 2, **dadurch gekennzeichnet, dass** der saure Zeolith ein Zeolith des H-ZSM-5-Typs ist.

4. Verfahren zur Herstellung einer Cyclopentylalkylether-Verbindung nach einem der Ansprüche 1 bis 3, **dadurch gekennzeichnet, dass** das Cyclopenten, das einen Substituenten haben kann, mit der durch Formel (2) dargestellten Alkoholverbindung in fließender Art umgesetzt wird.

5. Verfahren zur Herstellung einer Cyclopentylalkylether-Verbindung nach einem der Ansprüche 1 bis 4, **dadurch gekennzeichnet, dass** der saure Zeolith ein geformter Gegenstand ist.

## Revendications

1. Procédé de production d'un composé de type cyclopentylalkyléther représenté par la formule (1): R¹-O-R² , où R¹ représente un groupe alkyle ayant 1 à 10 atomes de carbone qui peut avoir un substituant ou un groupe cycloalkyle ayant 3 à 8 atomes de carbone qui peut avoir un substituant, et R² représente un groupe cyclopentyle qui peut avoir un substituant, dans lequel un cyclopentène qui peut avoir un substituent est mis à réagir avec un composé alcoolique représenté par la formule (2) : R¹OH, où R¹ représente le même que décrit ci-dessus, en présence d'une zéolithe acide ayant un rapport silice/alumine de 80 ou plus.

2. Procédé de production d'un composé de type cyclopentylalkyléther selon la revendication 1, **caractérisé en ce que** le composé de type cyclopentylalkyléther représenté par la formule (1) est un composé dans lequel R¹ dans la formule (1) est un groupe alkyle ayant 1 à 10 atomes de carbone.

3. Procédé de production d'un composé de type cyclopentylalkyléther selon la revendication 1 ou 2, **caractérisé en ce que** la zéolithe acide est une zéolithe de type H-ZSM-5.

4. Procédé de production d'un compose de type cyclopentylalkyléther selon l'une quelconque des revendications 1 à 3, **caractérisé en ce que** le cyclopentène qui peut avoir un substituant est mis à réagir avec le composé alcoolique représenté par la formule (2) d'une manière fluide.

5. Procédé de production d'un composé de type cyclopentylalkyléther selon l'une quelconque des revendications 1 à 4, **caractérisé en ce que** la zéolithe acide est un article moulé.
